## Europäisches Patentamt
## European Patent Office
## Office européen des brevets

(11) Publication number: **0 346 316**
**A2**

# EUROPEAN PATENT APPLICATION

(21) Application number: **89850190.3**

(22) Date of filing: **08.06.89**

(51) Int. Cl.4: **C 12 N 15/00**
A 61 K 39/21, A 61 K 39/395,
C 12 N 5/00, G 01 N 33/569,
G 01 N 33/68

(30) Priority: **10.06.88 SE 8802176**

(43) Date of publication of application:
**13.12.89 Bulletin 89/50**

(84) Designated Contracting States:
**AT BE CH DE FR GB IT LI NL SE**

(71) Applicant: **KabiGen AB**
**S-112 87 Stockholm (SE)**

(72) Inventor: **Gidlund, Magnus**
**Norrtullsgatan 26**
**S-113 45 Stockholm (SE)**

**Lake, Mats**
**Bodalsvägen 39**
**S-181 36 Lidingö (SE)**

**Löwenadler, Björn**
**Karlsholmsvägen 3F**
**S-183 64 Täby (SE)**

**Wigzell, Hans**
**Oscar Bäckströmsvägen 11**
**S-126 54 Hägersten (SE)**

(74) Representative: **Henningsson, Gunnar et al**
**Bergling & Sundbergh AB Box 7645**
**S-103 94 Stockholm (SE)**

(54) **Fusion protein and its use.**

(57) Fusion protein comprising a protein fragment carrying proteolytic activity and a CD4 binding glycosylated fragment originating from HIV;
DNA-sequence encoding the fusion protein;
antibody directed against the fusion protein;
plasmid or vector carrying the DNA-sequence;
host cell harbouring such plasmsid or vector;
methods for determining HIV-antibodies and CD4; and
method for the purification of the fusion protein.

Fig. 1

**Description**

## Fusion protein and its use

The present invention relates to a fusion protein comprising a proteolytically active protein fragment and a CD4 binding fragment. The invention also covers antibodies directed against such fusion protein, plasmids or vectors carrying same and, furthermore, methods for determining or detecting the presence of antibodies or CD4 and a method for the purification of the fusion protein.

Acquired Immunodeficiency syndrom (AIDS) denotes a complicated disease with a number of different diagnoses including severe bacterial and viral infections, malignancies and neurological deterioration of the central nervous system, CNS, leading to a state of dementia(1.2).

The causative agent of AIDS is the human immunodeficiency virus (HIV, formerly HTLV-III, LAV, ARV) (3.4). The virus belongs to the lenti virus group within the retrovirus family. HIV virus consists of at least two strains, HIV-1 and HIV-2, in which several isolated have been reported (5). A high degree of variability exists between the isolates especially in the gene encoding the gp160 protein. The gp160 protein is after synthesis cleaved into a transmembrane protein gp41 and gp120 localized in the outer membrane of the virus. The virus contains genetic information in the form of RNA, thus requiring a reverse transcriptase to complete a replicating round. The infectious cycle starts with a binding of the virus to the corresponding cell receptor CD4 localized on the outer membrane of the infectable cell (6) through the gp120, env(envelope), glycoprotein. The CD4 protein has shown to be present on preferentially subpopulation of human T-lymphocytes of the "Helper" type, Monocytes/macrophages, brain derived cells and in a low frequence on B lymphocytes (2.7.) The virus is then penetrating the outer membrane of the cell, through a mechanism involving "coated vesicles" and the infection is progressing through a RNA/DNA duplication followed by proviral integration into the cell host genome. After the integration the virus is activated by at large still unresolved mechanisms, and new virus particles are produced leading to the expression of gp120 on the cell surface of the infected cell, followed by syncytia formation, virus budding and cell death (7). The progression of the disease is therefore believed to be caused by the selective and subsequent elimination of cells within the immune system and CNS resulting both in an immunocompromised state and damages within CNS.

At present the disease is diagnosed by detection of soluble HIV antigen or antibodies against HIV in the serum from patients. This is done using a conventional solid-phase ELISA technique, based upon serum reaction against solubilized HIV antigens or synthetic peptides to detect antibodies or a capture anti HIV antibody to detect soluble ag. Problems which arise in these tests are primarily inability to detect antibodies against inter and intra strain variability within the HIV virus group. Neither do the tests allow the determination of antibodies relevant for prognosis or staging of the disease. Some existing evidence suggests that the antibody repertoire shown in patients is correlated to the actual disease (8) and that these in fact may play an important role in the body defence against the disease.

The main object of the present invention is to provide a new fusion protein, the use of which eliminates or at least greatly reduces the disadvantages associated with the prior art as outlined above, such as in connection with diagnosing AIDS.

Another object of the invention is to provide a DNA-sequence enabling expression of such fusion protein.

Yet another object is to provide methods for determining the presence of antibodies against HIV or the presence of CD4 in solution or expressed on the surface of mammalian cells.

Still another object is to provide a method for facile purification of the fusion protein of this invention.

The present invention in its basic aspect thus resides in a fusion protein comprising a protein fragment carrying proteolytic activity and a CD4 binding glycosylated fragment originating from HIV. Said proteolytically active protein fragment is preferably a t-PA fragment. Such fusion protein is suitably produced by expression in eucaryotic cells, thus allowing proper processing, e.g. glycosylation and folding, to occur. The production of the relevant fragments of the fusion protein is greatly facilitated by fusion between the peptide fragment of interest at the DNA level with a known sequence derived from the tissue plasmidian activator, abbreviated t-PA, and then expressed in a eucaryotic expression system, particularly of a mammalian nature. Primarily the invention thus provides for production of glycosylated peptides. The invention in its basic aspect is useful in many applications, as will be further outlined below.

In a preferred embodiment of the fusion protein of the invention the t-PA fragment comprises kringle 2 and the proteolytic portion of the t-PA molecule. The t-PA fragment may be glycosylated, but may also be non-glycosylated depending on its use. The non-glycosylated t-PA fragment will be useful in instances when non-interaction with the glycosylation of the CD4 binding fragment is desired.

It is preferred in accordance with the invention that the CD4 binding fragment of the fusion protein originates from HIV-envelope, such as HIV-gp120.

The invention also covers DNA-sequences encoding the fusion protein as described above, and such DNA-sequence preferably comprises the signal sequence of t-PA and possibly also the leader sequence of t-PA.

Within the scope of the invention there are also included antibodies directed against the fusion protein as described above or against the CD4 binding fragment of the fusion protein.

Furthermore, the invention covers plasmids or vectors carrying such DNA-sequence and host cells harbouring such plasmid or vector. The host cell is preferably a eucaryotic cell, such as a mammalian cell.

According to other aspects of the invention there is provided a method for determining the presence of antibodies against HIV in a specimen and also a method for detecting the presence of CD4 in solution or expressed on the surface of mammalian cells. Such methods comprise:
contacting a HIV-containing specimen or a specimen containing mammalian cells subject to investigation with a fusion protein comprising a t-PA fragment carrying proteolytic activity, and a CD4 binding glycosylated fragment originating from HIV. Such contact results in interaction between the antibodies or the CD4 of said cells and the fusion protein. The product resulting from the interaction is then analyzed using the proteolytic activity of the product of interaction. The method enables quantitative evaluation of the contents of antibodies or the CD4 contents of the cells subject to analysis.

According to yet another aspect of the invention there is provided a method for the purification of the fusion protein as described above. Such method comprises the steps:

   a) immobilizing antibodies as outlined above to an insoluble carrier;

   b) contacting a material containing said fusion protein with said immobilized antibodies resulting in interaction between same; and

   c) eluating the fusion protein from the carrier.

The elution is preferably made by lowering the pH.

The following is an outline of the different applications of using the fusion protein according to the invention.

a) Detection methods for serum bound antibodies against the envelope protein (gp120) of the Human Immunodeficiency (HIV) virus.

The detection method is based on the interaction between an active protein fragment from gp120 with maintained binding properties to the cellular receptor of the HIV virus CD4, and a proteolytically active fragment of t-PA. The binding between the gp120 fragment and matrix associated CD4 can be estimated by determining the quantity of bound gp120 fragment. Such determinations can be made either by measurement of the fusion/production component t-PA, or any other marker/indicator linked to the CD4 binding fragment of gp120.

If antibodies against the CD4 binding fragments from gp120 are present, such antibodies will prevent the binding of the fragment to CD4. The presence of such antibodies unequivocally proves the presence of a HIV mediated disease (AIDS).

b) Detection method for soluble CD4 binding proteins.

In analogy with the above, any protein having affinity for CD4 subsequently reacts with CD4 and prevents the binding of the t-PA gp120 fusion protein to the CD4. The quantity of soluble CD4 will therefore be correlated to the amount of measured fusion protein.

c) Detection method for cells expressing CD4.

Cells which express the CD4 protein will bind the t-PA/gp120 fusion protein and could therefore be assessed quantatively and qualitatively by measuring the amount of bound fusion protein.

d) Antigen for the generation of antibodies.

The fragments which are being produced contain antigenic structures extracted from the primary amino acid sequence as well as secondary and tertiary antigenic epitopes generated by the folding of the protein and the posttranslational glycosylation scheme occuring in eucaryotic cells. Immunisation protocols with fragment of the above nature will therefore generate antibodies against these structures. In the case of antibodies against the CD4 binding regions of gp120 such mixture of antibodies will be efficient in preventing the virus binding to infectable cells and thus impose a state of sterilizing immunity, or block in the progression of infection.

The basic structures of a glycoprotein are determined by the information given in the sequence of DNA encoding the glycoprotein in question. After the primary transcript, secondary and tertiary modifications occur including folding and glycosylation. In eucaryotic cells this mechanism is poorly understood, but it is clear that part of the information to direct such secondary processing and signals for secretion are given in the primary sequence of the DNA, mRNA and protein molecules during the synthesis. If only a part of a glycoprotein is to be made, especially if this part has a defined biological function, it is likely that important information concerning the secondary processing is missing.

This invention utilizes part of tissue Plasminogen activator (t-PA) as a fusion partner at the DNA-RNA and protein level and thus provides signals for the secondary processing and secretion. Furthermore, since the fragment may be labile/insoluble or display target areas for recognition and further fragmentation by proteolytic enzymes the physical linkage to t-PA provides stability, solubility and protects the fragment from further degradation.

The invention to use t-PA as a vehicle for the production of glycosylated fragments/peptides allows the subsequent analysis of the levels of protein produced by determination of the proteolytic activity in the t-PA part of the fusion molecule. Furthermore, the purification of the fusion protein could be made according to the antigenic nature of the t-PA molecule using specific matrix bound antibodies and conventional immunosorbent technique(s).

Another advantage of the invention is that it can utilize as a fusion partner a modified t-PA gene without

3

genetically encoded sites for glycosylation. This leads to the possibility of using this vehicle for production and analysis of sites and sequences of carbohydrates linked to a protein for determination of its specific biological function (e.g. ligand-receptor interaction, immunogenicity).

The present invention makes use of the interaction between gp120 and CD4. This interaction is physically strong but could be inhibited by antibodies against gp120 if CD4 is expressed on cells. The data generated suggest that this mode of measurement is more sensitive in terms of earlier detection time as well as the amount of antibody required to read a positive signal. Based on this finding the invention resides in the select expression of a part of gp120 involved in the CD4 binding region. This fragment contains conserved domains within the HIV-group (10), and by removal of nonbinding, non-conserved domains, which might "block" antibodies reacting against the CD4 binding sites, selection is made for antibodies with reactivity common to all HIV viruses. The fragments also contain possible glycosylation sites, allowing detection of anti-carbohydrate antibodies. Furthermore, the regions in question are likely to contain regions against which reacting antibodies might be clinically important. By linkage of the CD4 binding property to t-PA we have a possibility to directly quantitate the portion of the fusion protein engaged in the binding to CD4 by virtue of the proteolytic activity expressed by t-PA (11).

Cells within the body express cell surface bound protein molecules. In some cases there is a link between the presence of a cell surface molecule either to a certain cell type or to a function expressed by a set of cells. The CD4 marker is a well known marker preferentially expressed by a subpopulation of T cells known as Helper cells (6), a fraction of monocytes/macrophages (7) and some brain derived cells. The function of the CD4 molecule is still not fully understood but it serves as an important marker to the indicated cell types. Furthermore, in patient CD4 expressing cells it is an important prognostic tool. Using this invention, the number of CD4 expressing cells and number of CD4 receptors on one cell can be estimated by determining the binding of the fusion protein between t-PA and the CD4 binding region of gp120. Another similar aspect of the invention is that the same reaction can be used to detect soluble, non cell bound, CD4.

The Immune system has an inherent capacity to generate antibodies against epitopes determined by the primary, secondary and tertiary structures expressed in an antigenic molecule. Each type of antibody (i.e. originating from one clone of antibody producing cells) will react against one such epitope. If a protein fragment is used instead of a whole protein molecule for induction of antibody mediated immunity it is important to maintain the original properties of the fragment, such as glycosylation.

This invention allows the production of protein fragments from original antigenic molecules, through a mechanism involving eucaryotic secondary processing, thus imposing a high degree of homology between the original molecule and produced fragment thereof. Such fragment can, if used as an immunogen, induce the generation of antibodies which interact with structures homologous to the original molecule and against structures which by sterical hindrance, or by other means, including immunologically supressive epitopes (12), prevent the formation of antibodies against the epitope in the native configuration.

In the case that the fusion protein made between t-PA and CD4 binding region of the gp120 molecule would be used as an immunogenic molecule, either as a whole molecule, or with gp120 as a purified fragment, such immunogen will induce the formation of antibodies against structures present on the original molecule and against epitopes not usually exposed for the above reasons. Such antibodies alone or in combination with antibodies generated against the native molecule will thus be of importance to prevent binding of the virus to infectable cells via the CD4 receptor, thus preventing the infection mediated by HIV.

In summary, the invention is thus based on the construction of hybrid/fusion molecules between part of the t-PA molecule retaining proteolytic activity and protein fragments extracted from functional CD4 binding regions within the whole protein molecule from which the DNA is derived. The fusion is made at the DNA level, and by expression of the fused gene in a system allowing secondary processing, including folding and glycosylation, the produced fragment will maintain properties originating from the original molecule, and will in some instances, impose novel properties, including immunogenic potential and ability to react with antibodies. Thus, the invention will provide:

a) a rapid detection system based on the proteolytic activity of the t-PA fusion partner,

b) a purification system based upon immunosorbent techniques using properties expressed in the t-PA protein,

c) a system which allows the determination of function and position of biologically active protein carbohydrate substitutions,

d) a system which allows the production of a glycoprotein fragment extracted from a HIV DNA sequence coding for CD4 binding fragments for the purpose of generation of classification of antibodies for disease detection, protection or prevention.

EXAMPLE 1

Construction of a fusion protein gene consisting of the CD4 binding region of gp120, the cleavage site between gp120 and gp41, 5 N-terminal gp41 aa and kringle 2 plus the protease region of human tissue plasminogen activator.

Plasmid p17, containing bases 5820 to 8926 of the HIV virus genome was digested with Dral and Alul. A 524 bp fragment, encoding amino acids 343 to 516 of HIV envelope protein gp160, was purified by agarose electrophoresis and cloned into the small site of pUC19.

One recombinant plasmid harboring the 524 bp insert with the 5' Dral site oriented towards the Kpni site of the pUC19 multilinker was designated pKGE1000 and used for the further constructions.

Two synthetic oligonucleotides containing flanking EcoR1 and Kpn1 sites as well as an internal BamH1 site

```
5´AATTCGGATCCGGGTAC-3´

  3´-GCCTAGGCC-5´
```

for in frame fusion as described later were synthesized.

The synthetic linkers were ligated with EcoR1/Kpn1 digested pKGE1000 to yield a plasmid designated pKGE1001. In this plasmid the sequence coding for the CD4 binding region of gp160, contained within amino acid 343 to 516 (10), can be obtained on a 540 bp BamH1 fragment.

A gene encoding human tissue plasminogen activator lacking amino acids 6-173 has been extensively described in patent application 8702562-3 incorporated herein for reference. Apart from the 167 aa deletion the following amino acid residues were changed by synthetic oligonucleotides or site directed mutagenesis.

Asn-184 ---> Gln

Asn-177 ---> Ser

Lys-277 ---> Val

The modified t-PA encoded by this gene is designated k2(Gln)P (VaL).

The exemplary t-PA modification utilized here is similar to k2(Gln)P(Val) with the following exceptions:

Lys-277 is not modified

Asn-448 ---> Gln

Subsequently this modification is designated k2(Gln)P(Gln). One feature of the modified t-PA molecule encoded by this gene is that it lacks completely sites for asparagine linked glycosylation.

Furthermore it contains a BamH1 cleavage site immediately prior to the sequence coding for kringle 2 of t-PA. By insertion of the 540 bp BamH1 fragment of gp120 from pKGE1001 into this BamH1 site an in frame fusion between the CD4 binding region of gp120 and a modified form of t-PA is genera ted.

A plasmid containing part of this modified t-PA gene on a Sal1 Xmal fragment cloned into Sal1/Xmal cleaved pUC19 was digested with BamH1 and subsequently ligated with the 540 bp BamH1 fragment encoding the CD4 binding region of gp120, the cleaved site between gp120 and gp41 and 5 aa of gp41. A plasmid containing the BamH1 fragment inserted into the BamH1 site in the right orientation was designated pKGE1002.

pKGE1002 encodes the 35 aa t-PA signal peptide followed by the first 5 aa of the mature t-PA protein. Next follows aa 174-177 of t-PA (due to deletion of the EGF-, Finger- and, kringle 1 domains), three aa originating from the linker sequence and aa 343 to 516 of gp160. The fragment encodes apart from the CD4 binding domain of gp120, also the cleavage site between gp120 and gp41 and 5 amino acids of gp41. This feature makes the present gene fusion highly useful for production of peptides in eukaryotic systems were this cleavage site is utilized. Finally in the junction between aa 516 of gp160 and aa 178 of t-PA there are 3 aa originating from the pUC19 sequence. Together this gives a mature protein of 539 aa with N-terminal amino acids from t-PA/linker followed by 174 aa of gp160 and 353 aa of pUC19/t-PA sequence. The 350 amino acids encoding the C-terminal portion of t-PA spans the whole kringle 2 region and the complete protease domain.

For expression in mammalian cells two different eukaryotic expression vectors were constructed.

(i) For expression in COS cells a vector utilizing the SV40 late promoter was constructed.

pKGE1002 was digested with Sal1/Apa1. The Sal1/Apa1 fragment encoding the gp120 t-PA fusion was ligated with a vector consisting of pBR322, the SV40 late promoter, a modified t-PA gene (encoding K2(Gln)P(Gln) as well as 3′ regulatory signals from SV40, digested with Sal1/Apa1.

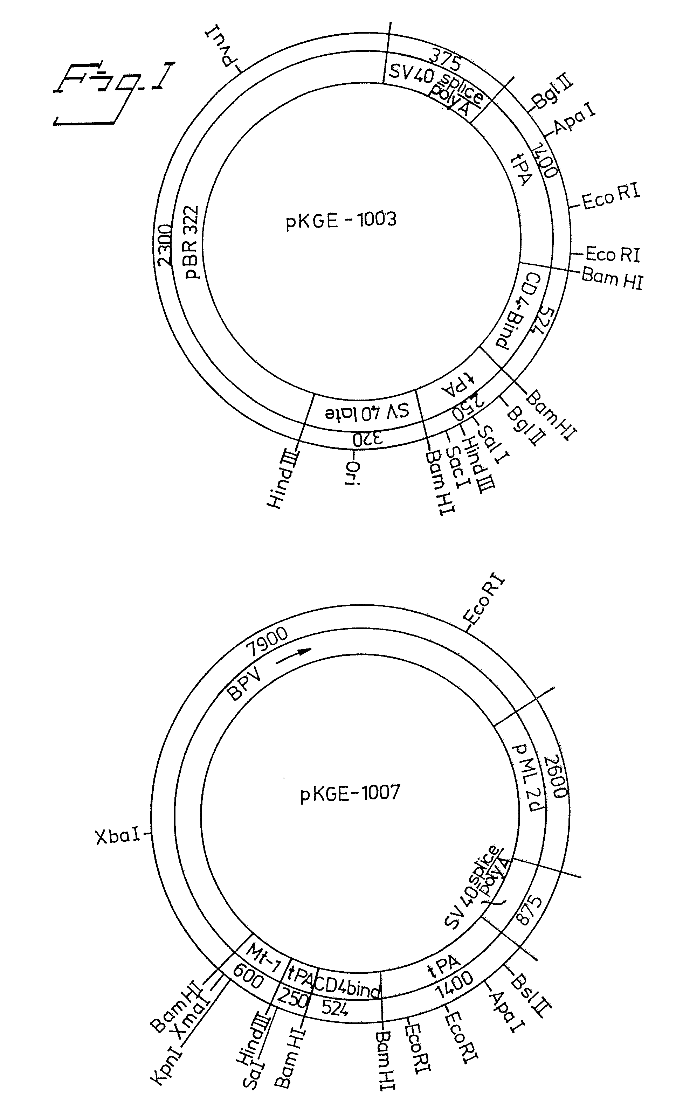

The resulting plasmid, pKGE1003 (see Fig. 1) contains the gene encoding the described gp120 t-PA fusion under transcriptional control of the SV40 late promoter. Down stream of the gene is a fragment from SV40 containing a splice site and polyadenylation signal.

(ii) For expression in rodent cells the gene segment encoding the gp120 t-PA fusion was inserted into expression vector pKGE113 described in Patent Application No. 8702562-3 incorporated herein for reference.

Briefly this plasmid consists of the whole genome of bovine papilloma virus (BPV) opened at its unique BamH1 site. Into this site is inserted the bacterial vector pML2d, the strong constitutive promoter mouse metallothionin-1, a modified t-PA gene followed by a splice and polyadenylation signal from SV40. By a three fragment ligation, the t-PA gene modified as in pKGE113 can be exchanged by the t-PA/gp120 fusion described here.

The gp120 sequence and most of the t-PA coding sequence is contained within a Sal1-Apa1 fragment. A Sal1-Xba1 fragment contains the MT-1 promoter and part of the BPV genome. Finally, a Xba1-Apa1 contains the rest of the BPV genome, pML2d, SV40 splice and polyadenylation signal and the remaining part of t-PA. By ligation of the three fragments, plasmid pKGE1004 was obtained.

EXAMPLE 2

Expression of gp120/t-PA in mammalian cells.

Plasmid pKGE1004 was transfected into hamster lung fibroblast cell line DON (ATCC CCL 16) in conjunction with a neomycin resistance gene. Cell clones growing in selective media containing G418 were subsequently isolated as individual cell isolates and expanded in 24 well plates. 48 of these clones were propagated and when confluence was reached the cell supernatant was analysed for fibrinolytic activity. Typical results are shown below:

TABLE 1

Fibrinolytic activity of isolated cell clones harbouring transfected cDNA encoding a fusion protein between Human t-PA/HIV gp120 fusion protein.

| Cell clone (A) | Fibrinolytic activity (B(radius mm)) |
|---|---|
| 1-5, 7-10 | 0 |
| 6 | 2 |
| 13 | 4 |
| 27 | 5 |
| 36 | 2 |

A = when cell clones were confluent 2ml of production media, containing 5 times lower concentration of Fetal Calf sera as ordinary culture media, was added and cells were further cultured for 24 hrs after which supernatants were assayed as in
B = assay performed using fibrinogen embedded Agar plates.

Result determined after 24 hrs.

Clones having the highest production values in the above test were subcultured in 250 ml flask which after confluence were expanded into roller flask. After confluence the media in the roller flask were substituted with production media as above and media was then harvested every third day during the culturing period. The supernatant was purified in the following ways:
1) The supernatant was clarified by centrifugation at 500xg. After centrifugation the medium was incubated with CNBr-Sepharose (Pharmacia, Sweden) immobilized (method provided with the purchaser) antibodies (a kind gift from Dr. Brandt, KabiVitrum AB, Sweden) directed against the proteolytically active part of human t-PA molecule. After batchwise adsorption the gel was recovered through consecutive centrifugations and the remaining gel was transferred to a column and washed with phosphate buffered saline (PBS) containing 0.01% Tween 20 and 0.25 M KSCN. Elution was performed with PBS-Tween containing 3M KSCN. A typical elution pattern is shown in Table 2:

TABLE 2

Elution profile from an anti-t-PA immunosorbent
adsorbed with medium from selected cell clones
producing a t-PA/gp120 fusion protein.

| Fraction after addition of elution buffer (A) | Fibrinolytic activity (B) |
|---|---|
| 1 | 0 |
| 2 | 0 |
| 3 | 2 |
| 4 | 5 |
| 5 | 4 |
| 6 | 3 |
| 7 | 2 |
| 8 | 1 |
| 9 | 0 |

A = Immunosorbent was batchwise adsorbed,
washed, transferred to a column, further washed
and then eluted. Fraction indicates fraction after
the elution buffer was added.
B = as in Table 1. 2 mm equals a t-PA content
equivalent to 500 ng of intact t-PA molecule.

2) Medium from cells was prepared as in 1). The medium was then adsorbed to a column consisting of antibodies from HIV, selected for reactivity against regions within the CD4 binding domain of gp120, immobilised on CNBr activate sepharose and further processed as in 1). A typical elution profile is shown below:

TABLE 3

Elution profile from an anti-HIV gp120 column
adsorbed with medium from cells producing a
t-PA/gp120 fusion protein.

| Fraction (A) | | Fibrinolytic activity (B) |
|---|---|---|
| Input | | 7 |
| passed through | | 0 |
| | 1 | 0 |
| | 2 | 2 |
| | 3 | 1 |
| | 4 | 1 |
| | 5 | 0 |

A = As in Tables 1 and 2 with the addition of the
input and passed through fraction, indicating a
complete adsorption of the fusion protein to the
column.
B = as in Tables 1 and 2.

The cells were cultured in the presence of tritiated (3-H) glucosamine and Mannose to label the carbohydrates on the produced peptide. Since the t-PA fusion partner is devoid of possible glycosylation sites any indication of the presence of carbohydrates emanates from the HIV part of the fusion protein. A typical result from such an elution is shown below:

TABLE 4

Elution profile from an anti-t-PA immunosorbent
column adsorbed with medium from cells
producing radioactively labelled carbohydrate
substituted t-PA/gp120 fusion protein

| Fraction (A) | Fibrinolytic activity (B) | glycosylation (C) |
|---|---|---|
| 1 | 0 | 150 |
| 2 | 0 | 160 |
| 3 | 1 | 535 |
| 4 | 5 | 2035 |
| 5 | 3 | 1135 |
| 6 | 2 | 765 |
| 7 | 1 | 560 |
| 8 | 0 | 460 |

A = as in Table 1 and 2.
B = as in Table 1 and 2.
C = measured radioactivity, derived from
incorporated glucosamine and Mannose, in the
samples assayed for fibrinolytic activity.

Material purified as in Table 1 was analyzed on a 12% SDS polyacrylamide gel by a fibrin overlay prepared as in Tables 1-5 and molecular weights determined. Method given in (Granelli-Piperno and Reich.J.Exp.Med. 148. 223. 1978. Zones appearing in the overlay were determined according to molecular weights. A typical experiment is shown below.

TABLE 5

Determination of molecular weights of fusion
protein between t-PA and gp120 eluted from an
anti-t-PA column.

| Protein (A) | Molecular weight (B) |
|---|---|
| Whole t-PA molecule | 68 |
| t-PA part of fusion protein alone | 40 |
| Fusion protein | 33 and 70-75 |

A = Whole t-PA molecule and t-PA variant protein
from the t-PA part of the t-PA/gp120 fusion
protein was kindly provided by Dr. G. Pohl,
KabiGen AB. Fusion protein prepared as in Table
2.
B = Kilo Dalton.

We could thus conclude that the protein which was made comprised of two species one consisting of a glycosylated full length protein and two additional fragments comprising the proteolytically active t-PA part and a hypothetical fragment derived from the HIV part. This fragment could however be detected by immunofluorescence using anti-HIV antisera

EXAMPLE 3

Construction of a fusion protein between the CD4 binding region of gp120 and modified t-PA, lacking the cleavage site between gp120 and gp41.

In order to get a stable fusion protein containing the CD4 binding region of gp120 and kringle 2 and the protease domain of t-PA, the cleavage site between gp120 and gp41 was removed according to the following scheme.

A unique StyI site in pKGE1002 cuts the DNA encoding gp120 about 11 amino acids upstream of the cleavage site between gp120 and gp41. By removing the DNA between the StyI site and the BamH1 site proceeding kringle 2 of t-PA in pKGE1002 and introducing a synthetic StyI-BamH1 linker there, plasmid pKGE1006 was produced.

5′-CAAGGCAAAGG-3′        Styl-BamH1-linker

   3′-CGTTTCCCTAG-5′

In pKGE1006 the DNA sequence encoding the cleavage site, the five first amino acids of gp41 and the 9 last amino acids of gp120 are removed, thus generating a gene segment encoding a fusion protein lacking two potential tryptic cleavage sites between gp120 and gp41.

For expression in mammalian cells the Sal1-Apa1 fragment of pKGE1006 spanning most of the coding region for this modified fusion protein was ligated with two other fragments containing the mouse MT-1 promoter, the BPV genome, pML2d and part of t-PA and 3′ processing signals from SV40, as described in Example 1.

This generates plasmid pKGE1007 (see Fig. 1) which contains a gene encoding amino acid 343 to 502 of HIV gp120 fused to kringle 2 and the protease domain of t-PA. This fusion lacks two potential tryptic cleavage sites between the CD4 binding region of gp120 and the t-PA regions.

## EXAMPLE 4

Expression in Mammalian cells.

The plasmid pKGE1007 was transfected into mouse C-127 cells (ATCC CRL 1616). Techniques for selection and further growing and processing of the media, including in part purification, was similar to Example 2. One additional experiment was performed. The transmembrane part CD4, being the receptor for HIV was produced by recombinant technology (kindly provided by Drs. P. Lind and Mona Sydow, KagiGen AB) and purified on an immunosorbent column using immobilized T4.2 antibodies, known to react with the HIV env binding part of CD4 (Dalgleish et al. Nature 312, 763, 1984). After purification the CD4 protein was analyzed on SDS-PAGE gel and shows one single band at 42 Kilo Dalton, with selective gp120 binding properties (protein purified, analyzed and kindly supplied by Drs. A.Nygren and M. Jansson, Department of Immunology, Karolinska Institute). In the following experiment supernatant from cells producing t-PA/gp120 fusion protein was allowed to adsorb to a column containing immobilized CD4 and then subsequently eluted using the conditions used in Example 1 Table 2. One typical result is shown in the Table below.

TABLE 1

Analysis of CD4 binding properties of the
t-PA/gp120 fusion protein

| Supernatant adsorbed with gel containing (A) | Recovered fibrinolytic activity(B) |
|---|---|
| Non substituted | 0 |
| Anti-t-PA | 15 |
| CD4 | 8 |

A= Proteins were coupled on CNBr activated Sepharose (Pharmacia, Sweden) and used as recommended by the manufacturer.
B= as in Example 1 Table 1.

## DEPOSITS

Microorganisms, recombinant DNA molecules and the two gp120 t-PA coding sequences of this invention have been deposited in the culture collection of "Deutsche Sammlung von Mikroorganismen", Marschroder Weg 1B, D-3300 Braunschweig, Germany on June 9, 1988 and have been identified there as:
A: E.coli DH5 (pKGE1003) accession number: DSM 4662
B: E.coli DH5 (pKGE1007) accession number: DSM 4663

References

1. Navia et.al Ann. Neurol. 19, 525 (1986)
2. Chiodi et.al J. Virol. 61, 1244 (1987)
3. Barre-Sinoussi et.al Science 220, 668 (1983)
4. Gallo, R.C. Science 224, 500 (1985)
5. Wong-Stahl et.al Nature 317, 395 (1985)
6. Dalgleish et.al Nature 312, 763 (1984)

Asjö, B., et.al Virology 157, 359 (1987)

8. Ljunggren, K., et.al J. Immunol., 139, 2263 (1987)

9. Lundin, K. J. Immunol., 97, 93 (1987)

10. Laskey et.al Cell 50, 975 (1987)

11. Pohl et.al Biochem. 23, 3701 (1987)

12. Herzenberg, L.A., et.al Ann. Rev. Immmunol. 1, 609 (1987)

## Claims

1. Fusion protein comprising a protein fragment carrying proteolytic activity and a CD4 binding glycosylated fragment originating from HIV.

2. Fusion protein according to claim 1, wherein said protein fragment is a t-PA fragment.

3. Fusion protein according to claim 1 or 2, wherein said t-PA fragment comprises kringle 2 and the proteolytic portion of the t-PA molecule.

4. Fusion protein according to claim 1, 2 or 3, wherein said t-PA fragment is glycosylated.

5. Fusion protein according to claim 1, 2 or 3, wherein said t-PA fragment is non-glycosylated.

6. Fusion protein according to any preceding claim, wherein said CD4 binding fragment originates from HIV-envelope.

7. Fusion protein according to claim 6, wherein said fragment originates from HIV-gp120.

8. DNA-sequence encoding the fusion protein of any of the preceding claims.

9. DNA-sequence according to claim 8, comprising the signal sequence of t-PA.

10. DNA-sequence according to claim 8 or 9, comprising also the leader sequence of t-PA.

11. Antibody directed against the fusion protein of any of claims 1 or 7 or against a CD4 binding fragment of said protein.

12. A plasmid or vector carrying the DNA sequence of any of claims 8 to 10.

13. A host cell harbouring the plasmid or vector according to claim 12.

14. A host cell according to claim 13 which is a eucaryotic cell.

15. A host cell according to claim 14 which is a mammalian cell.

16. A method for determining the presence of antibodies against HIV in a specimen, said antibodies reacting with regions within the CD4 binding fragment derived from the primary sequence of HIV-envelope, comprising the steps:

    a) contacting said specimen with a fusion protein comprising a t-PA fragment carrying proteolytic activity, and a CD4 binding glycosylated fragment originating from HIV, said contact resulting in interaction between the antibodies of said specimen and said fusion protein;

    b) analyzing the product resulting from said interaction using the proteolytic activity of the product; and

    c) evaluating the contents of antibodies of said specimen.

17. A method for detecting the presence of CD4 in solution or expressed on the surface of mammalian cells, comprising the steps:

    a) contacting a specimen containing such cells with a fusion protein comprising a t-PA fragment carrying proteolytic activity, and a CD4 binding glycosylated fragment originating from HIV, said contact resulting in interaction between the CD4 of said cells and said fusion protein;

    b) analyzing the product resulting from said interaction using the proteolytic activity of the product; and

    c) evaluating the CD4 contents of said cells.

18. A method according to claim 16 or 17, wherein said t-PA fragment comprises kringle 2 and the proteolytic protein of the t-PA molecule.

19. A method according to claim 16, 17 or 18, wherein said CD4 binding fragment originates from HIV-envelope.

20. A method according to claim 19, wherein said fragment originates from HIV-gp120.

21. A method for the purification of the fusion protein according to any of claims 1 to 7, comprising the steps:

    a) immobilizing antibodies according to claim 9 to an insoluble carrier;

    b) contacting a material containing said fusion protein with said immobilized antibodies resulting in interaction between same; and

    c) eluating the fusion protein from the carrier.

22. A method according to claim 21, wherein the elution is made by lowering the pH.

Fig. 1

EP 0 346 316 A2